# EUROPEAN PATENT APPLICATION

(11) **EP 0 887 078 A1**
(43) Date of publication of application: **30.12.1998**
(21) Application number: 98304713.5
(22) Date of filing: 15.06.1998
(51) Int. Cl.: A61K 31/195

(54) **Use of L or DL-threo-DOPS for preventing and treating the consequences of cerebral ischemia**

(30) Priority: 15.06.1997 JP 172963/97
(71) Applicant: Nishino, Katsuhiro, Akita-shi, Akita 010-0821 (JP); Sumitomo Pharmaceuticals Company, Limited, Osaka 541-8510 (JP)
(72) Inventor: Nishino, Katsuhiro, Akita-shi, Akita 010-0821 (JP)
(74) Representative: Cresswell, Thomas Anthony

(57) **Abstract**

Use of L- or DL-threo-DOPS or a pharmaceutically acceptable acid addition salt thereof in the manufacture of a medicament for preventing or treating neurological deficits or neuronal death induced by cerebral ischemia.

## Description

The present invention relates to use of a medicament for the prevention or treating at a superacute phase against neurological deficits or neuronal death induced by cerebral infarction, subarachnoid hemorrhage, brain injury, and other relevant cerebral ischemia.

L-threo-DOPS (Droxidopa) is a central norepinephrine precursor and is known as an active ingredient for norepinephrine activated nerve function improving agents. Clinically, L-threo-DOPS is used to improve freezing and orthostatic dizziness in Parkinson's disease (stage 3 in the degree of severity according to Yahr) and also to improve orthostatic hypotension, syncope and orthostatic dizziness in familial amyloid polyneuropathy or Shy-Drager Syndrome. The safety of L-threo-DOPS has been confirmed experimentally and clinically.

As shown in Patent Application Laying Open No. 8-20535, a restorative neuropharmacological agent for motor, consciousness, and speech disturbance originating from central nervous system disorders containing L-threo-DOPS as an active ingredient was developed by the present inventor.

Conventionally, only a few pharmacological modalities have been established as agents for preventing neurological deficits or neuronal death induced by cerebral ischemia such as cerebral infarction and for the treatment at a superacute phase thereof. Most of agents were employed in vessels, for example, thrombolysis of cerebrovascular thrombi and emboli has been attempted in cerebral infarction. For thrombolytics, however, it is not easy to select therapeutic window, etc., to determine clinical cases for whom they are effective. In addition, there has been such a problem that thrombolytics not rarely cause serious adverse effects that may lead patient's death such as intracerebral hemorrhage.

On the other hand, neuroprotective agent in cerebral ischemia has not been established especially with direct action onto neuron.

The present invention has been achieved paying attention to the above-mentioned problems. It is an object of the present invention to provide use of a medicament for preventing neurological deficits or neuronal death induced by cerebral ischemia or treating at a superacute phase thereof, which is highly safe in terms of adverse effects as compared to thrombolytic agents.

In order to achieve the above-mentioned object, an agent for the prevention of neurological deficits or neuronal death and the treatment at a superacute phase thereof, 1) by cerebral ischemia or cerebral infarct, 2) by subarachnoid hemorrhage, 3) by brain injury. and 4) by cerebral ischemia due to surgery, especially neurosurgery and cardiovascular surgery according to the present invention are characterized in that they comprise L- or DL-threo-DOPS or their pharmaceutically acceptable acid-addition-salt as an active ingredient.

The expression "neurological deficits or neuronal death" used in this specification includes all neurological deficit or neuronal death induced by cerebral ischemia, such as consciousness disturbance, aphasia, motor paralysis, agnosia, apraxia, dementia. and visual disturbance. "Cerebral ischemia" includes cerebral ischemia accurred during surgery, angiospasm in subarachnoid hemorrhage, intracerebral hemorrhage, cerebral infarction, brain injury, and any other relevant pathological conditions. The expression "agent for the treatment at a superacute phase" used in the specification means a drug whose administration is initiated within short period of time, such as several hours, following the onset of neuronal ischemic insult. The agent for the treatment at a superacute phase according to the present invention is preferably started to be administered as soon as possible following the onset, and more preferably started to be administered immediately or within 6 hours after the onset at normal body temperature, if possible.

L-threo-DOPS (Droxidopa) is (-)-(2S, 3R)-2-amino-3-hydroxy-3-(3, 4-dihydroxyphenyl) propionic acid (according to the JAN nomenclature) or (-)-threo-3-(3, 4-dihydroxyphenyl) L-serine (according to the INN nomenclature), the structural formula of which is shown below.

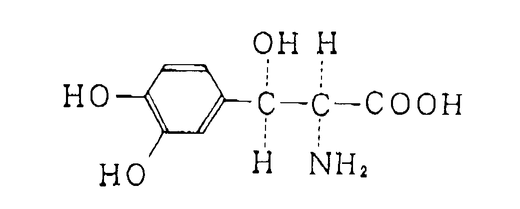

L-threo-DOPS, whose molecular formula is C₉H₁₁NO₆, is white or light brown crystals or crystalline powder with no taste or odor. Droxidopa dissolves only slightly in water, and dissolves very little in ether, ethanol and glacial acetic acid. It is difficult to measure a clear melting point or decomposition point for L-threo-DOPS. L-threo-DOPS shows some changes when the temperature is raised to the vicinity of 220 °C, starts melting at 225 °C, and turns into a black liquid at around 230 °C. The pKa of L-threo-DOPS is 7.88, measured by the titration method. DL-threo-DOPS, which contains 50% L-threo-DOPS, can also be used in this invention. Hereafter, L-threo-DOPS and DL-threo-DOPS are generically denoted as "threo-DOPS".

In this invention, threo-DOPS can be used in a pharmaceutically acceptable acid-addition-salt form as well. For example, inorganic acids such as hydrochloric acid, hydrobromic acid and sulfuric acid arid organic acids such as fumaric acid, citric acid, tartaric acid and succinic acid can be used to form an acid-addition-salt.

Threo-DOPS can be manufactured by means of prior art methods such as those described in USP4562263 and USP4480109.

Some of the prior art pharmacological features of threo-DOPS are follows: (1) it is directly converted to I-norepinephrine by the action of the aromatic L-amino acid decarboxylase which is widely distributed in a living body, and thus has an effect of replenishing norepinephrine, (2) it passes through the blood-brain barrier into the brain, (3) it specifically recovers norepinephrine activated nerve functions which have decreased in the central and peripheral nervous system, and (4) it shows various actions via the adrenaline receptors in various tissues.

Threo-DOPS was found to be effective in ameliorating motor paralysis and motor aphasia in the chronic phase of cerebral stroke due to subarachnoid hemorrhage, cerebral infarction, and intracerebral hemorrhage and post-traumatic brain injury wherein physical therapy alone cannot provide improvement, and particularly effective in increasing the recovery rate. It was also found to be effective in recovering from consciousness and activity disturbances in patients with cerebral stroke not accompanied by brainstem disorders.

The present invention is based on the finding that threo-DOPS is also effective in preventing neurological deficits or neuronal death induced by cerebral ischemia and in treating at a superacute phase thereof. The agent for the prevention and for the treatment at a superacute phase according to the present invention is effective in ameliorating consciousness disturbance, motor paralysis, and other neurological deficit or neuronal death induced by cerebral ischemia due to subarachnoid hemorrhage, intracerebral hemorrahage, cerebral infarction, brain injury, surgery, and other cerebral ischemia and in decreasing the rate of aggravation thereof. Specific pathological conditions to which the agent for the prevention and for the treatment at a superacute phase according to the present invention is actually applicable clinically and the effects of administration are:

(1) Prevention and alleviation of neurological deficits or neuronal death upon recurrence of brain embolism as in cases with atrial fibrillation; (2) prevention of recurrence of cerebral infarction. regression of infarction lesions upon recurrence. and prevention and alleviation of neurological deficits or neuronal death upon recurrence; (3) regression of infarction lesions and prevention and alleviation of neurological deficits or neuronal death due to cerebral infarction by administration in a acute phase of cerebral infarction (within several hours after onset); (4) prevention and alleviation of neurological deficits or neuronal death induced by transient blockade of blood flow in neck clipping of cerebral aneurysm upon subarachnoid hemorrhage, carotid endoarterectomy, or bypass surgery and prevention of occurrence of cerebral infarction induced by the same circumstances; (5) prevention and alleviation of neurological deficits or neuronal death due to cerebrovascular spasm in subarachnoid hemorrhage and prevention of occurrence of cerebral infarction due to the same; (6) prevention and alleviation of neurological deficits or neuronal death and prevention of occurrence of cerebral infarction during and after surgery using a artificial heart-lung apparatus; and (7) brain-protection effect and antilethal effect in patients with severe intracranial hypertension or deep coma with mydriasis in cases of subarachnoid hemmorage, cerebral stroke, or brain injury.

The mechanism of development of these pharmacological effects is considered to be as follows: Threo-DOPS directly acts on neurons to exert an effect to protect against neuronal death due to cerebral ischemia, an antilethal effect, and an anti-edema effect due to excessive polarization of neuronal membrane potential caused by an increase in Na-K-ATPase activity with noradrenaline.

This neuroprotective agent which contains threo-DOPS as an active ingredient can be in any form including capsules, tablets, confection, pills, parvule, suppository, solution and ampules. This neuroprotective agent which contains threo-DOPS as an active ingredient can contain fillers. expanders, binders, dissolution retardants, surfactants, adsorbents, lubricants, coloring agents. perfumes, preservatives, etc. Such preparations can be manufactured following a typical common method. This unique neuroprotective agent can also contain other pharmaceutically active ingredients as well. A prior art norepinephrine activated nerve function improving agent which has L-threo-DOPS as an active ingredient is commercially available under the name "DOPS" (manufactured and distributed in Japan by Sumitomo Pharmaceuticals Company, Limited ).

In particular, the neuroprotective agent according to this invention should preferably be used together with a peripheral decarboxylase inhibitor such as benserazide (hereafter referred to as "BSZ") or carbidopa to promote transfer of threo-DOPS into the brain. To use them together, separate preparations of threo-DOPS and of a peripheral decarboxylase inhibitor can be administered either at the same time or at different times, or a mixture of them can be administered. BSZ can be manufactured using a prior art method.

For the administration method of the neuroprotective agent according to this invention, depending on the form it takes, oral administration. rectal administration, nasal administration, intravenous administration, hypodermic administration, intramuscular administration, etc. are possible, of which oral administration is preferable.

The amount of oral administration of the neuroprotective agent according to this invention is 60-1,200 mg droxidopa for regular adults per day, preferably 100-900 mg, and more preferably 100-400 mg. When a peripheral decarboxylase inhibitor is contained, the amount of the peripheral decarboxylase inhibitor contained is empirically 1-10% of the amount of droxidopa, preferably 2.5-7.5% and more preferably about 5%. For example 5% is particularly preferable for BSZ. However, depending on the age, weight, symptoms, and past history etc, of the subject to whom the medication is administered, the amount of administration of droxidopa and the peripheral decarboxylase inhibitor can be changed as appropriate. In clinical examples, administration of droxidopa alone has shown sufficient efficacy.

Preparations according to this invention are described below by referring to examples. However, this invention is not limited to those examples.

### Example 1: Preparing capsules

200 weight parts of droxidopa, 167 weight parts of an excipient and 3 weight parts of a lubricant (this is something which gives a smooth and glossy surface to the preparation) are homogeneously mixed, and empty capsules are filled with this mixture in such a way that each capsule contains 200 mg of droxidopa. A capsule preparation is thus obtained.

### Example 2: Preparing capsules

100 weight parts of droxidopa, 168 weight parts of an excipient and 2 weight parts of a lubricant are homogeneously mixed, and empty capsules are filled with this mixture in such a way that each capsule contains 100 mg of droxidopa. A capsule preparation is thus obtained.

The excipient for Examples 1 and 2 described above is chosen from among lactose, white sugar. glucose, D-mannitol, potato starch, corn starch, wheat starch, calcium carbonate, calcium sulfate, anhydrous calcium phosphate, sodium bicarbonate, crystalline cellulose, a mixture of these, etc. The lubricant is chosen from among magnesium stearate, calcium stearate, talc, etc.

Figure 1 is a graph showing the volumes of cerebral infarction lesions when the drug was administered before occlusion in the animals experiencing occlusion of the middle cerebral artery for 1 hour in the experiment.

Figure 2 is a graph showing the volumes of cerebral infarction lesions when the drug was administered 1 hour after occlusion in the animals experiencing occlusion of the middle cerebral artery for 1 hour in the experiment.

Figure 3 is a graph showing the volumes of cerebral infarction lesions when the drug was administered 1 hour after occlusion in the animals experiencing occlusion of the middle cerebral artery for 24 hour in the experiment.

Figure 4 is a graph showing the hematocrit levels of the models used in the experiment.

In order to determine whether the agent prevents neurological deficits or neuronal death induced by cerebral ischemia, animal experiments with MC occlusion model were carried out.

### Experiment

### Outline of the Methods

Transient cerebral ischemia was introduced in the middle cerebral artery (MC) occulusion model Wistar rats under halothane anesthesia and it was studied whether L-threo-DOPS influences on the size of infarction. In the experiment, the agent was administered before (group 1) and one hour after occlusion (group 2) in rats in which MC was occluded for 1 hour, then reperfused for 23 hours. In other experiment, the agent administered one hour after occlusion in rats in which MC occulusion remained for 24 hours (group 3). For each group, the model rats were further divided into three groups: a vehicle control group, a group receiving BSZ, and a group receiving L-threo-D0PS + BSZ. BSZ was found not to elevate blood pressure at the dose administered.

### Model Preparation Method

Males Slc-Wistar rats aged 12-13 weeks and weighing 270-320 g were employed. MC was occluded by a plug method and reperfused. A rat was placed under anesthesia with 2% halothane and underwent midline incision of the neck. Then, the right external carotid artery was dissected with care. A nylon plug with silicon coating (#4-0, 17.5 mm in length) was inserted from a starting point of the external carotid artery to the internal carotid artery side to occlude an MC initial portion. Immediately after completion of the surgery, anesthesia was terminated. The rat was kept vigil until the time of reestablishment of reflow in occluded MC territory. Core temperature was maintained by placing the rat on a warming pad from the beginning of surgery to the time awakening from anesthesia, Successful MC occlusion was judged using left hemiplegia as an index. Rats not showing hemiplegia were excluded from the experiment groups. In the 1-hour MC occlusion group, a plug was removed under halothane anesthesia one hour after occlusion to restart blood fl ow. In the 24-hour MC occlusion group, a plug was removed under halothane anesthesia 24 hour after occlusion to restart blood flow.

### Drug Administration

L-threo-DOPS (400 mg/kg) and/or BSZ (2 mg/kg) were administered intraperitoneally 30 minutes before 1-hour MC occlusion, one hour after 1-hour MC occlusion, and one hour after 24-hour MC occlusion.

L-threo-DOPS was dissolved in 2 ml of a 0.5% methylcellulose solution and BSZ was dissolved in 1 ml of saline for administration.

### Calculation of Cerebral Infarction Lesion

Twenty-four hours after reperfusion of MC, the brain was perfused with a physiological saline solution containing heparin under anesthesia with pentobarbiturate and then the whole brain was isolated after decapitation. The isolated brain was cut into coronal sections with a thickness of 1 mm using a tissue chopper and the sections were incubated in a physiological saline solution containing 2% of 2,3,5-triphenyltetrazolium-C1 for 15 minutes for color development of non-infarction regions. After color development. tissue was fixed in 10% formaline solution and a volume of infarction was measured using an NIH image program.

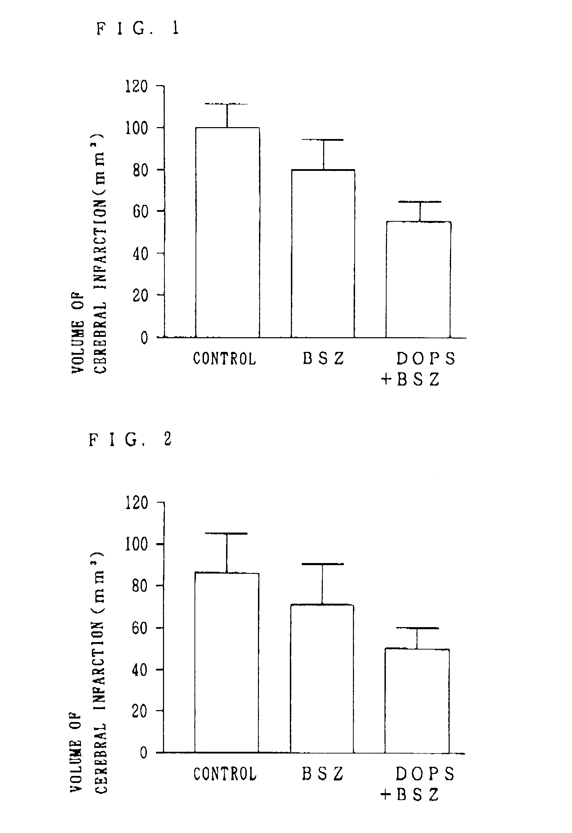

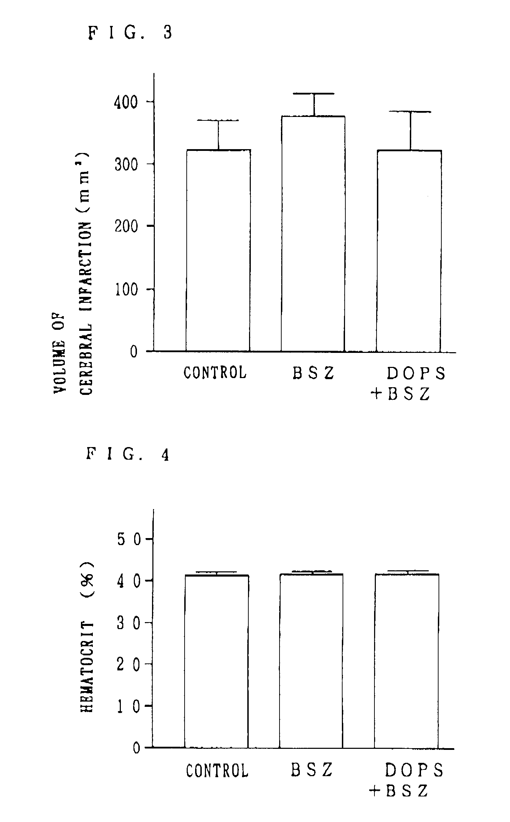

The results of the experiment are shown in Figures 1-3. As shown in Figures 1 and 2, administration of L-threo-D0PS before and one hour after MC occlusion, respectively, reduced the size of cerebral infarction caused by MC occlusion for 1 hour by about 40%, confirming an effect of threo-DOPS to regress cerebral infarction. As shown in Figure 3, however, no regression effect was observed for administration of complete cerebral infarction caused by MC occlusion for 24 hours. As shown in Figure 4, there was no significant differences in hematocrit level among the three groups.

In order further to examine the neuroprotective effect in cerebral ischemia indicated from the experiment, a clinical preliminary study was conducted.

### Clinical Case 1

### Clinical subject: A female aged 59 years old

### Diagnosis: Subarachnoid hemorrhage and intracerebral hemorrhage due to rupture of right middle cerebral artery aneurysm

Although radical surgery was performed on the second day after the onset, the patient exhibited coma (JCS(Japan coma scale): 30) and left hemiplegia (1/5) (Hunt & Kosnic Grade IV), neurological symptoms were not ameliorated. After the neck clipping surgery, 500 mg/day of "DOPS" (registered trademark. Sumitomo Pharmaceuticals Co., Ltd., Japan) was administered for 5 days. As a result, further aggravation of neuronal symptoms due to cerebral angiospasm was not observed, and not only improvement of consciousness but also alleviation of left hemiplegia (4/5) and improvement of skill movement such as knitting were observed three weeks later.

### Clinical Case 2

### Clinical subject: A female aged 69 years old

### Diagnosis: Subarachnoid hemorrhage due to rupture of the left internal carotid artery aneurysm

Neck clipping was conducted on the day of onset, when the patient's condition was Hunt & Kosnic Grade IV. From the third day after subarachnoid hemorrhage, 500 mg/day of "DOPS" (registered trademark) was administered for 5 days. Then, no aggravation of neuronal symptoms and CT findings of cerebral infarct were found. Eventually, the symptoms were ameliorated with a progress similar to that observed for clinical case 1.

### Clinical Case 3

### Clinical subject: A male aged 65 years old

### Diagnosis: Subarachnoid hemorrhage due to rupture of anterior communicating artery aneurysm

Neck clipping was conducted on the day of onset, when the patient's condition was Hunt & Kosnic Grade IV. Since consciousness disturbance (semicoma), hyperventilation, and reduced light reflex were observed on the sixth day after surgery, a diagnosis of impending cerebral herniation was established and 500 mg/day of "DOPS" (registered trademark) was administered for 5 days. During administration. no mydriasis indicating a progress of hernia was observed. Soon after discontinuation of administration, mydriasis was rapidly observed.

### Clinical Case 4

### Clinical subject: A male aged 60 years old

### Diagnosis: Right cerebral infarction (embolism) and atrial fibrillation

Left hemiplegia first appeared and consciousness disturbance (JCS:100) and mydriasis of the right eye were observed 2 days later. Immediately after that, external and internal decompressions was performed and 300 mg/day of "DOPS" (registered trademark) was simultaneously administered for 5 days. Although JCS was 200 (deep coma) three weeks after surgery, consciousness was improved and hemiplegia (2/5) was ameliorated 1 month later.

### Clinical Case 5

### Clinical subject: A male aged 51 years old

### Diagnosis: Cerebral contusion

From the time immediately after injury, the patient developed mydriasis in both eyes and deep coma due to impending herniation. Immediately, 500 mg/day of "DOPS" (registered trademark) was administered for 5 days. As a result, spontaneous respiration was maintained for 6 days.

### Clinical Case 6

### Clinical subject: A male aged 47 years old

### Diagnosis: Intracerebral hemorrhage

The patient developed deep coma (JCS: 200) and bilateral light reflex disappearance upon onset. Although 500 mg of "DOPS" (registered trademark) was administered 2 days after the onset. the drug was not effective.

### Clinical Case 7

### Clinical subject: A female aged 40 years old

### Diagnosis: Left acute subdural hemorrhage

The patient visited the hospital because of consciousness disturbance (JCS: 200), mydriasis of the left eye, and right light reflex disappearance 3 days after falling down and underwent emergent surgery. Although 500 mg of "DOPS" (registered trademark) was administered 12 hours after appearance of mydriasis of the left eye, the drug was not effective.

### Clinical Case 8

### Clinical subject; A female aged 70 years old

### Diagnosis: Subarachnoid hemorrhage due to rupture of left internal carotid artery aneurysm

Neck clipping was performed on the day of onset, when the patient's condition was Hunt & Kosnic grade II. On the tenth day after the onset, the patient developed consciousness disturbance (JCS: 100), right hemiplegia, and aphasia. Three hours after appearance of the symptoms, 500 mg of "DOPS" (registered trademark) was administered via the stomach tube. Administration at the same dose was continued for further 4 days. The patient showed recovery from 2 hours after administration and consciousness disturbance was ameliorated to JCS: 3 and right hemiplegia to 4/V twelve hours after first administration. In the CT findings then obtained, appearance of low-density area due to cerebrovascular spasm was slight. Two months after the onset, the Patient became to be able to walk alone.

Based on sequential neurological & neuroradiological evaluation in these clinical cases, it is found that neurological deficis or neuronal death induced by cerebral ischemia such as in subarachnoid hemorrhage. intracerebral hemorrhage, and cerebral infarction can be prevented, when L-threo-DOPS is administered immediately following the onset of neurological deficits or neuronal death induced by cerebral ischemia. Clinical cases 1 and 2 are understood to be the cases in whom administration before the initiation of cerebrovascular spasm can prevent these symptoms due to cerebral ischemia. In clinical cases 3 and 5, the rate of aggravation of neurological signs induced by a impending herniation was delayed following administration of L-threo-DOPS, and L-threo-DOPS is considered to suppress a progress of brainstem hernia. In clinical case 8, L-threo-DOPS was administered upon the appearance of signs due to cerebrovascular spasm, and rapid amelioration of the signs different from an usual progress was observed. In any case, it should be noted that preventive administration or administration in a superacute phase shortly after onset is critical to expect these effects. No adverse effects were observed in clinical cases 1-8.

Droxidopa has already been commercially available in Japan, and the following is reported regarding its safety.
(A) Acute toxicity (LD50) is shown in Table 1.

**TABLE 1**

| Acute toxicity results (LD50, mg/kg) | | | | | | | | |
|---|---|---|---|---|---|---|---|---|
| Administration via | Mice, ICR strain | | Rats, SD strain | | beagle dogs | | rhesus monkeys | |
| | Male | Female | Male | Female | Male | Female | Male | Female |
| Oral | >1000 | >10000 | >10000 | >10000 | >5000 | >5000 | >5000 | >5000 |
| Hypodermic | >10000 | >10000 | 84 | 95 | - | - | - | - |
| Intravenous | >100 | >100 | 16-20 | 19 | - | - | - | - |

As for general symptoms, reduced voluntary motions, deep breathing and bradypnea were observed with mice and rats, but no abnormal symptom was observed with dogs and rhesus monkeys.
(B) Subacute toxicity testing
60, 300, 1,500 mg/kg/day was orally administered to SD rats, 200, 600, 2,000 mg/kg/day was orally administered to beagle dogs, and 300. 1.000, 3,000 mg/kg/day was orally administered to rhesus monkeys consecutively for 3 months. As a result, for dogs and monkeys, no abnormality was observed in various observations and testing evaluations. For the rats, suppressed voluntary motions. necrosis of kidney uriniferous tubuli, necrosis of cardiac muscles, etc. were observed with 60 mg/kg/day and higher. Suppression of weight increases and such were observed with 300 mg/kg/day and higher. Drooling was observed with 1.500 mg/kg/day.
(C) Chronic toxicity testing
10. 30, 100, 300 mg/kg/day was orally administered to SD rats, and 125. 500, 2,000 mg/kg/day was orally administered to beagle dogs consecutively for 12 months. As a result, for dogs, no abnormality was observed in various observations and testing evaluations. For the rats, suppression of weight increases, an increase in thymus weight, necrosis of kidney uriniferous tubuli, degeneration of kidney glomeruli, etc. were observed with 30 mg/kg/day and higher, and drooling, myocarditis and calcification of cardiac muscles, etc. were observed with 100 mg/kg/day and higher.
(D) Procreation testing
(1) Pre-pregnancy and early pregnancy administration testing 60, 200, 600 mg/kg/day was orally administered to SD rats (male and female) consecutively. As a result, no influence was observed on the mating rate, conception rate, nidation number, embryo/neonate death, teratogenic actions or fetal development.
(2) Organ forming period administration testing
   60, 200, 600 mg/kg/day was orally administered to SD rats consecutively. As a result, lower body weights of fetuses and an increase in the occurrence of undulating ribs were observed with 200 mg/kg or higher. However, they were within the range in which they can be restored after birth. No other influence was observed.
   30, 100, 300 mg/kg/day was orally administered to rabbits consecutively. As a result, no influence on the rabbits was observed.
(3) Perinatal and lactation period administration testing
   60, 200, 600 mg/kg/day was orally administered to SD rats consecutively. As a result, shortening of the pregnancy period was observed with 600 mg/kg, and suppression of neonatal development after birth was observed with 60 mg/kg or higher. No other influence was observed.

(E) Antigenicity testing
Endodermoreaction, systemic anaphylaxis reaction, PCA reaction and intra-gel sedimentation reaction testing on guinea pigs (Hartley strain, male) yielded negative results.
(F) Mutagenicity testing
Back mutation testing using microorganisms, chromosome abnormality testing using cultured cells and micronucleus testing on mice were conducted and no mutagenicity was observed.

It is understood that although the present invention has been described in detail with respect to preferred embodiments thereof, various other embodiments and variations are possible to those skilled in the art which fall within the scope and spirit of the invention, and such other embodiments and variations are intended to be covered by the following claims.

## Claims

1. Use of L- or DL-threo-DOPS or a pharmaceutically acceptable acid addition salt thereof in the manufacture of a medicament for preventing or treating neurological deficits or neuronal death induced by cerebral ischemia.

2. Use according to claim 1 wherein the medicament is for treating neurological deficits or neuronal death induced by cerebral ischemia at a superacute phase.

3. Use according to claim 1 wherein the medicament is for preventing cerebral infarction.

4. Use according to claim 1 or 2 wherein the medicament is for treating cerebral infarction at a superacute phase.

5. Use according to claim 1 or 2 wherein the medicament is for treating cerebral ischemic sequel of subarachnoid hemorrhage or intracerebral hemorrhage.

6. Use according to claim 1 or 2 wherein the medicament is for treating traumatic brain injury at a superacute phase.

7. Use according to claim 1 wherein the medicament is for preventing neurological deficits or neuronal death induced by cerebral ischemia associated with surgery.

8. Use according to claim 1 or 2 wherein the medicament is for treating neurological deficits or neuronal death induced by cerebral ischemia associated with surgery at a superacute phase.

9. Use of L- or DL-threo-DOPS or a pharmaceutically acceptable acid addition salt thereof in the manufacture of a medicament for preventing or treating intracranial hypertension or cerebral edema.

10. Use according to any one of the preceding claims, wherein the medicament further comprises a peripheral decarboxylase inhibitor selected from benserazide and carbidopa.
